# EUROPÄISCHE PATENTSCHRIFT

(11) **EP 2 742 172 B1**
(45) Veröffentlichungstag und Bekanntmachung des Hinweises auf die Patenterteilung: **14.10.2015**
(21) Anmeldenummer: 12744004.8
(22) Anmeldetag: 10.08.2012
(51) Int. Cl.: C25B 3/02

(54) **VERFAHREN ZUR HERSTELLUNG VON VANILLIN DURCH ELEKTROCHEMISCHE OXIDATION VON WÄSSRIGEN LIGNIN-LÖSUNGEN BZW. SUSPENSIONEN**
METHOD FOR PRODUCING VANILLIN BY ELECTROCHEMICALLY OXIDIZING AQUEOUS LIGNIN SOLUTIONS OR SUSPENSIONS
PROCÉDÉ DE FABRICATION DE VANILLINE PAR OXYDATION ÉLECTROCHIMIQUE DE SOLUTIONS OU SUSPENSIONS AQUEUSES DE LIGNINE

(30) Priorität: 11.08.2011 EP 11177320; 04.07.2012 EP 12175005
(43) Veröffentlichungstag der Anmeldung: 18.06.2014
(73) Patentinhaber: BASF SE, 67056 Ludwigshafen (DE); Rheinische Friedrich-Wilhelms-Universität Bonn, 53113 Bonn (DE)
(72) Erfinder: STECKER, Florian, 68167 Mannheim (DE); MALKOWSKY, Itamar Michael, 67346 Speyer (DE); FISCHER, Andreas, 64646 Heppenheim (DE); WALDVOGEL, Siegfried R., 55218 Ingelheim (DE); REGENBRECHT, Carolin, 55116 Mainz (DE)
(74) Vertreter: Reitstötter Kinzebach
(86) Internationale Anmeldenummer: PCT/EP2012/065642
(87) Internationale Veröffentlichungsnummer: WO 2013/021040

(56) Entgegenhaltungen:
- EP-A1- 0 882 814
- PARPOT P ET AL: "Biomass conversion: attempted electrooxidation of lignin for vanillin production", JOURNAL OF APPLIED ELECTROCHEMISTRY, KLUWER ACADEMIC PUBLISHERS, DO, Bd. 30, Nr. 6, 1. Juni 2000 (2000-06-01), Seiten 727-731, XP002545692, ISSN: 1572-8838, DOI: 10.1023/A:1004003613883
- TIAN M ET AL: "A novel approach for lignin modification and degradation", ELECTROCHEMISTRY COMMUNICATIONS, ELSEVIER, AMSTERDAM, NL, Bd. 12, Nr. 4, 1. April 2010 (2010-04-01), Seiten 527-530, XP026977282, ISSN: 1388-2481, DOI: 10.1016/J.ELECOM.2010.01.035 [gefunden am 2010-01-28]
- CARMEN Z. SMITH ET AL: "Electro-organic reactions. Part 60[1]. The electro-oxidative conversion at laboratory scale of a lignosulfonate into vanillin in an FM01 filter press flow reactor: preparative and mechanistic aspects", JOURNAL OF APPLIED ELECTROCHEMISTRY, Bd. 41, Nr. 4, 26. November 2010 (2010-11-26), Seiten 363-375, XP055044214, ISSN: 0021-891X, DOI: 10.1007/s10800-010-0245-0
- B Moodley ET AL: "The electro-oxidation of lignin in Sappi Saiccor dissolving pulp mill effluent", Water SA (Online), 37 (1), 31. Januar 2011 (2011-01-31), Seiten 33-40, XP055044895, http://www.scielo.org.za/scielo.php?script =sci_arttext&pid=S1816-79502011000100006&l ng=en&nrm=iso ISSN: 1816-7950 Gefunden im Internet: URL:http://www.ajol.info/index.php/wsa/art icle/viewFile/64104/51902 [gefunden am 2012-11-20]
- PETR ZUMAN AND ELINORE B. RUPP: "Electrochemical Investigations of Alkaline Cleavage of Lignin Under Mild Conditions", COLLECTION OF CZECHOSLOVAK CHEMICAL COMMUNICATIONS, [Online] Bd. 66, Nr. 7, 2001, Seiten 1125-1139, XP002687571, DOI: 10.1135/cccc20011125 Gefunden im Internet: URL:http://cccc.uochb.cas.cz/66/7/1125/> [gefunden am 2012-11-20]
- VERA L. PARDINI ET AL: "Anodic cleavage of lignin model dimers in methanol", TETRAHEDRON, Bd. 48, Nr. 35, 1. Januar 1992 (1992-01-01), Seiten 7221-7228, XP055044781, ISSN: 0040-4020, DOI: 10.1016/S0040-4020(01)88262-4
- John C. Wozniak: "Preparation and Reactions of Diels-Alder Adducts of Lignin-Derived Quinones, Doctor's Dissertation, June 1988", , Juni 1988 (1988-06), Seiten 1-173, XP055044921, The Institute of Paper Chemistry, Appleton, Wisconsin Gefunden im Internet: URL:http://smartech.gatech.edu/bitstream/h andle/1853/5515/wozniak_jc.pdf?sequence=1 [gefunden am 2012-11-20]

## Beschreibung

Die Erfindung betrifft ein Verfahren zur Herstellung von Vanillin durch elektrochemische Oxidation einer wässrigen, ligninhaltigen Suspension oder Lösung.

Lignine sind eine Gruppe dreidimensionaler, in der Zellwand von Pflanzen vorkommender Makromolekülen, die sich aus verschiedenen phenolischen Monomerbausteinen wie p-Cumarylalkohol, Coniferylalkohol und Sinapylalkohol zusammensetzen. Lignine werden beim Wachstum der Pflanzen in die pflanzliche Zellwand eingelagert und bewirken dadurch die Verholzung der Zelle (Lignifizierung). Etwa 20 % bis 30 % der Trockenmasse verholzter Pflanzen bestehen aus Ligninen. Neben Cellulose und Chitin sind Lignine somit die häufigsten organischen Verbindungen der Erde.

Lignin sowie ligninhaltige Substanzen wie Alkalilignin, Ligninsulfat oder Ligninsulfonat, fallen in verschiedenen industriellen Prozess wie der Papierherstellung in großer Menge als Nebenprodukt an. Die Gesamtproduktion ligninhaltiger Substanzen wird auf etwa 20 Milliarden Tonnen pro Jahr geschätzt. Lignin stellt somit einen wertvollen Rohstoff dar. Teile dieses Lignins werden mittlerweile weiter verwendet. Beispielsweise wird Alkalilignin, welches durch alkalische Behandlung der bei der Papierherstellung anfallenden Schwarzlauge herstellbar ist, in Nordamerika als Bindemittel für Pressplatten auf Holz und Cellulosebasis, als Dispergiermittel, zur Klärung von Zuckerlösungen, Stabilisierung von Asphaltemulsionen sowie Schaumstabilisierung verwendet. Der weitaus größte Teil des Abfall-Lignins wird jedoch durch Verbrennung als Energiespender z.B. für den Zellstoffprozess verwendet. Da es sich bei Lignin um einen aromatischen Wertstoff handelt, ist es neben seiner energetischen Verwertung wünschenswert Lignin in größerem Umfang zu weiteren Wertstoffen umzusetzen.

Vanillin, 4-Hydroxy-3-methoxybenzaldehyd ist ein synthetischer Aromastoff, der anstelle der teuren natürlichen Vanille in großem Umfang als Aromastoff für Schokolade, Süßwaren, Liköre, Backwaren und andere süße Lebensmittel sowie zur Herstellung von Vanillinzucker verwendet wird. Kleinere Mengen werden in Deodorantien, Parfüms, und zur Geschmacksverbesserung von Pharmazeutika und Vitaminpräparaten verwendet. Vanillin ist auch Zwischenprodukt bei der Synthese von verschiedenen Arzneimitteln wie z.B. L-Dopa, Methyldopa und Papaverin. Es besteht daher grundsätzliches Interesse an neuen wirtschaftlichen Verfahren zur Herstellung von Vanillin. Aufgrund seiner strukturellen Ähnlichkeit zu den Basisbausteinen des Lignins eignet sich der Aromastoff Vanillin als Zielmolekül für Synthesen ausgehend vom Lignin.

WO 87/03014 beschreibt ein Verfahren zur elektrochemischen Oxidiation von Lignin bei Temperaturen von vorzugsweise 170 bis 190 °C in wässrigen, stark alkalischen Lösungen unter Durchmischen während der Elektrolyse. Als Anoden werden vor allem Kupfer- oder Nickelelektroden eingesetzt. Als niedermolekulares Produkt wird ein komplexes Gemisch erhalten, das unter anderem Vanillinsäure (4-Hydroxy-3-methoxybenzoesäure), Vanillin, 4-Hydroxybenzaldehyd, 4-Hydroxyacetophenon und Acetovanillon (4-Hydroxy-3-methoxyacetophenon) sowie gegebenenfalls Phenol, Syringasäure (4-Hydroxy-3,5-dimethoxybenzoesäure) und Syringaldehyd (4-Hydroxy-3,5-dimethoxybenzaldehyd) enthält. In der Regel ist 4-Hydroxybenzoesäure das Hauptprodukt. Nur unter Verwendung von Nickelelektroden gelingt es Vanillin als Hauptprodukt der Elektrolyse zu gewinnen, wobei allerdings Temperaturen von 170°C und als Elektrolyt 3 M Natronlauge erforderlich sind. Die stark alkalischen Bedingungen und die hohen Temperaturen führen jedoch dazu, dass das bei der Oxidation gebildeten niedermolekularen Produkte Abbaureaktionen wie Überoxidation und Disproportionierung erleiden. Zudem sind die wässrigen alkalischen Lösungen unter den in WO 87/03014 beschriebenen Bedingungen stark korrosiv und führen zu einer Zerstörung der Elektrolysezelle und des Elektrodenmaterials. Diese Korrosionsprozesse bedingen einen nicht unbeträchtlichen Schwermetalleintrag in die erhaltenen Produkte, so dass diese auch nach Aufreinigung für die Lebensmittelindustrie nicht mehr geeignet sind.

C.Z. Smith et al. J. Appl. Electrochem. 2011, DOI 10.1007/s10800-010-0245-0 beschreiben ebenfalls Untersuchungen zur elektrochemischen Oxidation von Ligninsulfat zu Vanillin unter alkalischen Bedingungen an Nickelelektroden bei Temperaturen von 170°C. Als Elektrolysezelle wird eine Zelle mit Umlauf eingesetzt, bei der der Ligninsulfat-haltige Elektrolyt im Umlauf kontinuierlich durch eine zylindrische Elektrodenanordnung mit zentralem zylindrischem Nickelnetz als Kathode und einem die Kathode zylindrisch umgebenden Nickelnetz als Anode geleitet wird.

Die WO 2009/138368 beschreibt ein Verfahren zum elektrolytischen Abbau von Lignin, bei dem ein wässriger ligninhaltige Elektrolyt an einer Diamantelektrode oxidiert wird. Dabei wird unter anderem ein niedermolekulares Produkt gebildet, das etwa in gleichen Anteilen Vanillin zusammen mit anderen Hydroxybenzaldehyd-Derivaten wie Acetovanillin oder Guajakol enthält. Die Selektivität der Ligninoxidation bezüglich Vanillin ist somit gering. Wie eigene Untersuchungen der Erfinder zeigen, ist die Diamantelektrode den stark korrodierenden Bedingungen bei basischen pH-Werten während der Elektrolyse nicht gewachsen. Schon nach kurzer Zeit ist die Diamantelektrode stark geschädigt. Es ist somit notwendig die Elektrolyse im sauren pH-Bereich durchzuführen.

Parpot P. et al., Journal of Applied Electrochemistry 2000, DOI:10.1023/A:1004003613883, beschreiben den elektrochemischen oxidativen Abbau von Kraft-Lignin in Batch- und Durchflusszellen an Pt, Au, Ni, Cu, DSA-O₂ und PbO₂ Anoden.

Tian M. et al., Electrochemistry Communications 2010, DOI:10.1016/J.ELECOM.2010.01.035 beschreiben die photochemische und elektrochemische Oxidation von Kraft-Lignin mit Ta₂O₅-IrO₂ Beschichtung auf TiO₂ Nanoröhrenanordnung.

EP 0882814 beschreibt die elektrochemische Delignifizierung von wässriger Pulpe mit ligninhaltigem Material

J Aufgabe der vorliegenden Erfindung ist die Bereitstellung eines Verfahrens, das die Herstellung von Vanillin durch elektrochemische Oxidation von Lignin oder ligninhaltigen Substanzen in guten Ausbeuten und mit hoher Selektivität bezüglich der Bildung von Vanillin erlaubt. Weiterhin soll das Verfahren unter Bedingungen durchführbar sein, die weniger korrosiv als die Bedingungen des Standes der Technik sind und die eingesetzten Elektroden weniger stark angreifen. Insbesondere soll das Vanillin in einer Form erhalten werden, die einem Einsatz als Aromastoff in der Lebensmittelindustrie nicht entgegenstehen.

Diese und weitere Aufgaben werden durch das im Folgenden beschriebene Verfahren zur elektrochemischen Oxidation von ligninhaltigen wässrigen Stoffströmen gelöst, bei dem als Anode eine Silberelektrode verwendet wird.

Die vorliegende Erfindung betrifft somit ein Verfahren zur Herstellung von Vanillin, umfassend eine elektrochemische Oxidation einer wässrigen, ligninhaltigen Suspension oder Lösung an einer Anode, wobei als Anode eine Silberelektrode verwendet wird.

Ein weiterer Gegenstand der vorliegenden Erfindung ist die Verwendung des Vanillins, welches durch das erfindungsgemäße Verfahren hergestellt wurde, als Aromastoff in der Nahrungsmittelindustrie.

Das erfindungsgemäße Verfahren ist mit einer Reihe von Vorteilen verbunden. So führen die verwendeten Elektrodenmaterialien zu einer signifikanten Selektivitätssteigerung. Diese hohe Selektivität kann überraschenderweise auch bei vergleichsweise niedriger Temperatur von bis zu 100°C erzielt werden. Zudem erweisen sich die erfindungsgemäß verwendeten Anodenmaterialien als äußerst widerstandsfähig gegenüber den korrosiven Reaktionsbedingungen und, anders als bei den Verfahren des Standes der Technik, findet keine oder keine nennenswerte Korrosion statt.

Unter ligninhaltigen wässrigen Lösungen oder Suspensionen wird hier und im Folgenden eine wässrige Lösung oder Suspension verstanden, die Lignin oder Ligninderivate, beispielsweise Ligninsulfat, Ligninsulfonat, Kraft-Lignin, Alkalilignin oder Organosolv-Lignin oder Mischung davon, als Ligninbestandteil enthält. Bei der wässrigen Lösung bzw. Suspension kann es sich um eine wässrige Lösung bzw. Suspension handeln, die als Nebenprodukt bei einem technischen Verfahren wie der Papierstoff-, Zellstoff- oder Cellulose-Herstellung anfällt, z.B. Schwarzlauge, sowie die ligninhaltigen Abwasserströme aus dem Sulfit-Prozess, aus dem Sulfat-Prozess, aus dem Organocell- oder Organosolv-Prozess, aus dem ASAM-Verfahren, aus dem Kraft-Verfahren oder aus dem Natural-Pulping Verfahren. Bei der wässrigen Lösung bzw. Suspension kann es sich um eine wässrige Lösung bzw. Suspension handeln, die durch Auflösen eines Lignins oder Ligninderivats hergestellt wird, z.B. Ligninsulfat, Ligninsulfonat, Kraft-Lignin, Alkalignin oder Organosolv-Lignin, oder einem Lignin das bei einem technischen Verfahren wie der Papierstoff-, Zellstoff oder Cellulose-Herstellung anfällt, z.B. Lignin aus Schwarzlauge, aus dem Sulfit-Prozess, aus dem Sulfat-Prozess, aus dem Organocell- oder Organosolv-Prozess, aus dem ASAM-Verfahren, aus dem Kraft-Verfahren oder aus dem Natural-Pulping Verfahren.

In dem erfindungsgemäßen Verfahren wird ein wässriger, ligninhaltiger Elektrolyt, der Lignin oder eine ligninhaltige Substanz enthält und der in Form einer wässrigen Suspension oder Lösung vorliegt, einer elektrochemische Oxidation, d.h. einer Elektrolyse unterworfen. Hierbei findet an der Anode die Oxidation des enthaltenen Lignins oder des Ligninderivats statt. An der Kathode erfolgt typischerweise eine Reduktion des wässrigen Elektrolyten z.B. unter Bildung von Wasserstoff.

Im erfindungsgemäßen Verfahren kann als Anode grundsätzlich jede dem Fachmann bekannte Silberelektrode eingesetzt werden. Diese kann vollständig aus Silber oder einer silberhaltigen Legierung bestehen oder eine Trägerelektrode sein, die einen Träger aufweist, der mit Silber oder einer silberhaltigen Legierung beschichtet ist. Bei den als Anode verwendeten Elektroden kann es sich beispielsweise um Elektroden in Form von Streckmetallen, Netzen oder Blechen handeln.

Als silberhaltige Legierung können dem Fachmann bekannte silberhaltige Münzlegierungen eingesetzt werden. Diese enthalten neben Silber bevorzugt Kupfer, Nickel, Eisen oder Mischungen dieser Metalle. Zu nennen sind Kupfer-Silber, Nickel-Silber, Silber-Eisen und Kupfer-Nickel-Silber. Bevorzugte Silberlegierungen weisen typischerweise einen Silbergehalt von wenigstens 50 Gew.-% auf. Der Anteil der weiteren Silberbestandteile liegt typischerweise im Bereich von 1 bis 40 Gew.-%, insbesondere im Bereich von 5 bis 35 Gew.-%. Beispiele für derartige Silberlegierungen ist eine Legierung aus 90 Gew.-% Silber und 10 Gew.-% Nickel sowie Cuprosilber, bei dem es sich um Eine Legierung aus 72,5 Gew.-% Silber und 27,5 Gew.-% Kupfer handelt.

Bevorzugt wird als Anode eine Silberelektrode eingesetzt, in der Silber oder eine silberhaltige Legierung als Beschichtung auf einem elektrisch leitfähigen, von Silber verschiedenen Träger angeordnet ist. Die Dicke der Silberschicht beträgt dabei in der Regel weniger als 1 mm, z.B. 10 bis 300 µm, vorzugsweise 10 bis 100 µm.

Als Trägermaterialien für derartige mit Silber beschichtete Elektroden eignen sich elektrisch leitfähige Materialien wie Niob, Silizium, Wolfram, Titan, Siliziumcarbid, Tantal, Kupfer, Gold, Nickel, Eisen, Graphit, keramische Träger wie Titansuboxid oder silberhaltige Legierungen. Als Träger bevorzugt sind Metalle, insbesondere Metalle mit einem geringeren Standardpotential als Silber wie beispielsweise Eisen, Kupfer, Nickel oder Niob. Bevorzugt ist es, Träger in Form von Streckmetallen, Netzen oder Blechen einzusetzen, wobei die Träger insbesondere die zuvor genannten Materialien enthalten. Insbesondere bestehen diese Streckmetalle oder Bleche zu 50 Gew.-%, bevorzugt 75 Gew.-% insbesondere 95 Gew.-% bezogen auf das Gesamtgewicht des Trägers aus Eisen, Kupfer oder Nickel.

Als Kathode kann grundsätzlich jede dem Fachmann bekannte, für die Elektrolyse wässriger Systeme geeignete Elektrode eingesetzt werden. Da an der Kathode Reduktionsprozesse stattfinden und das Vanillin an der Anode oxidiert wird, ist bei Verwendung einer Schwermetall-Elektrode wie beispielsweise einer Nickelkathode die Belastung des Vanillins mit diesem Schwermetall so gering, dass das erhaltene Vanillin problemlos in der Nahrungsmittelindustrie eingesetzt werden kann. Dennoch ist es vorteilhaft keine Kathoden einzusetzen, die Nickel oder Blei enthalten. Vorzugsweise zeigen die Elektrodenmaterialien eine geringe Wasserstoffüberspannung. Bevorzugt sind dabei Elektroden, die ein Elektrodenmaterial ausgewählt unter Silber, Nickel, silberhaltigen Legierungen, RuOxTiOx-Mischoxid, platiniertem Titan, Platin, Edelstahl, Graphit oder Kohle enthalten. Insbesondere bevorzugt ist dabei ein Elektrodenmaterial ausgewählt unter Silber, platiniertem Titan, Nickel, Platin oder Edelstahl, vor allem Silber, Nickel und Platin. Besonders bevorzugt handelt es sich bei der Kathode um eine beschichtete Edelmetallelektrode. Als Edelmetallschicht kommen insbesondere Beschichtungen aus Silber oder Platin oder Legierungen, die im Wesentlichen, d.h. zu wenigstens 50 Gew.-%, aus Silber, Platin oder Mischungen davon bestehen, in Betracht. Die Dicke der Edelmetallschicht beträgt dabei in der Regel weniger als 1 mm, z.B. 10 bis 300 µm. Als Trägermaterialien für derartige mit Edelmetall beschichtete Elektroden eignen sich elektrisch leitfähige Materialien wie sie zuvor im Zusammenhang mit der Silberelektrode genannt wurden. Bevorzugt ist es, Träger in Form von Streckmetallen, Netzen oder Blechen einzusetzen, wobei die Träger insbesondere die zuvor genannten Materialien enthalten. Insbesondere bestehen diese Streckmetalle oder Bleche zu 50 Gew.-%, bevorzugt 75 Gew.-% insbesondere 95 Gew.-% bezogen auf das Gesamtgewicht des Trägers aus Eisen oder Kupfer.

Die Anordnung von Anode und Kathode ist nicht beschränkt und umfasst beispielsweise Anordnungen von planaren Gittern und/oder Platten, die auch in Form mehrerer, alternierend gepolter Stapel angeordnet sein können und zylindrische Anordnungen von zylindrisch geformten Netzen, Gittern oder Rohren, die auch in Form mehrerer, alternierend gepolter Zylinder angeordnet sein können.

Zur Erzielung optimaler Raum-Zeit-Ausbeuten sind dem Fachmann verschiedene Elektrodengeometrien bekannt. Vorteilhaft sind eine bipolare Anordnung mehrerer Elektroden, eine Anordnung, in der eine stabförmige Anode von einer zylindrischen Kathode umfasst wird, oder eine Anordnung, in der sowohl die Kathode als auch die Anode aus einem Drahtnetz bestehen und diese Drahtnetze aufeinander gelegt und zylindrisch aufgerollt wurden.

In einer Ausführungsform der Erfindung sind die Anode und Kathode durch einen Separator voneinander getrennt. Grundsätzlich sind als Separatoren alle in Elektrolysezellen üblicherweise eingesetzten Separatoren geeignet. Der Separator ist typischerweise ein zwischen den Elektroden angeordnetes poröses Flächengebilde, z.B. ein Gitter, Netz, Gewebe oder Vlies, aus einem elektrisch nicht-leitenden Material, das unter den Elektrolysebedingungen inert ist, z.B. ein Kunststoffmaterial, insbesondere ein Teflon-Material oder ein mit Teflon beschichtetes Kunststoffmaterial.

Für die Elektrolyse können jegliche dem Fachmann bekannte Elektrolysezellen eingesetzt werden, wie geteilte oder ungeteilte Durchflusszelle, Kapillarspaltzelle oder Plattenstapelzelle. Besonders bevorzugt ist die ungeteilte Durchflusszelle, z.B. eine Durchflusszelle mit Umlauf, bei der der Elektrolyt kontinuierlich im Umlauf an den Elektroden vorbeigeführt wird. Das Verfahren kann mit gutem Erfolg sowohl diskontinuierlich als auch kontinuierlich durchgeführt werden.

Das erfindungsgemäße Verfahren kann ebenfalls im industriellen Maßstab durchgeführt werden. Entsprechende Elektrolysezellen sind dem Fachmann bekannt. Alle Ausführungen dieser Erfindung beziehen sich sowohl auf den Labor- wie auf den industriellen Maßstab.

In einer bevorzugten Ausführungsform der Erfindung wird der Inhalt der Elektrolysezelle durchmischt. Für diese Durchmischung des Zelleninhalts können jegliche dem Fachmann bekannte mechanische Rührer eingesetzt werden. Die Verwendung von anderen Durchmischungsmethoden wie der Einsatz von Ultraturrax, Ultraschall oder Einstrahldüsen ist ebenfalls bevorzugt.

Durch Anlegen der Elektrolysespannung an den Anoden und den Kathoden wird durch den Elektrolyten elektrischer Strom geführt. Um Nebenreaktionen wie Überoxidation und Knallgasbildung zu vermeiden, wird man in der Regel eine Stromdichte von 1000 mA/cm², insbesondere 100 mA/cm², nicht überschreiten. Die Stromdichten, bei denen man das Verfahren durchführt, betragen im Allgemeinen 1 bis 1000 mA/cm², bevorzugt 1 bis 100 mA/cm². Besonders bevorzugt wird das erfindungsgemäße Verfahren bei Stromdichten zwischen 1 und 50 mA/cm² durchgeführt.

Die Gesamtdauer der Elektrolyse hängt naturgemäß von der Elektrolysezelle, den verwendeten Elektroden und der Stromdichte ab. Eine optimale Dauer kann der Fachmann durch Routineversuche, z.B. durch Probennahme während der Elektrolyse ermitteln.

Um einen Belag auf den Elektroden zu vermeiden, kann die Polung in kurzen Zeitabständen gewechselt werden. Die Umpolung kann in einem Intervall von 30 Sekunden bis 10 Minuten erfolgen, bevorzugt ist ein Intervall von 30 Sekunden bis 2 Minuten. Hierfür ist es zweckmäßig, dass Anode und Kathode aus dem gleichen Material bestehen.

Aus dem Stand der Technik bekannte Verfahren müssen häufig bei hohen Drücken und bei Temperaturen weit über 100 °C durchgeführt werden. Die stellt besondere Anforderungen an die Elektrolysezelle, da diese für Überdruck ausgelegt sein muss. Weiterhin leiden sowohl die Elektrolysezelle als auch die Elektroden unter den korrosiven Bedingungen, die sich bei einer hohen Temperatur einstellen. In dem erfindungsgemäßen Verfahren ist es nicht notwendig bei hohen Drücken und Temperaturen zu arbeiten.

Die Elektrolyse wird gemäß dem erfindungsgemäßen Verfahren in der Regel bei einer Temperatur in einem Bereich von 0 bis 100 °C, bevorzugt 50 bis 95 °C, insbesondere 75 bis 90°C durchgeführt.

In dem erfindungsgemäßen Verfahren wird die Elektrolyse in der Regel bei einem Druck unterhalb 2000 kPa, bevorzugt unterhalb 1000 kPa, insbesondere unterhalb 150 kPa z.B. im Bereich von 50 bis 1000 kPa, insbesondere 80 bis 150 kPa durchgeführt. Besonders bevorzugt ist es das erfindungsgemäße Verfahren bei einem Druck im Bereich des Atmosphärendrucks (101 ± 20 kPa) durchzuführen.

In einer besonders bevorzugten Ausführungsform wird das erfindungsgemäße Verfahren bei 80°C bis 85°C und im Bereich des Atmosphärendrucks (101 ± 20 kPa) durchgeführt.

Die wässrige, ligninhaltige Suspension oder Lösung enthält im Allgemeinen 0,5 bis 30 Gew.-%, vorzugsweise 1 bis 15 Gew.-%, insbesondere 1 bis 10 Gew.-% Lignin bezogen auf das Gesamtgewicht der wässrigen, ligninhaltigen Suspension oder Lösung.

Bei allen Prozessen der Papier-, Zellstoff oder Celluloseherstellung, fallen ligninhaltige Abwasserströme an. Diese können als wässrige, ligninhaltige Suspension oder Lösung im erfindungsgemäßen Verfahren verwendet werden. Die Abwässerströme des Sulfitverfahrens zur Papierherstellung enthalten Lignin häufig als Ligninsulfonsäure. Ligninsulfonsäure kann in dem erfindungsgemäßen Verfahren direkt eingesetzt oder zuerst alkalisch hydrolysiert werden. Im Sulfat- oder Kraftverfahren fallen ligninhaltige Abwasserströme z.B. in Form von Schwarzlauge an. Im Organocell-Verfahren, das aufgrund seiner Umweltfreundlichkeit in Zukunft weitere Bedeutung erlangen wird, fällt das Lignin als Organosolv-Lignin an. Ligninsulfonsäurehaltige oder Organosolv-Ligninhaltige Abwasserströme sowie Schwarzlauge eignen sich in besonderer Weise als wässrige, ligninhaltige Suspension oder Lösung für das erfindungsgemäße Verfahren.

Alternativ können die wässrigen, ligninhaltigen Suspensionen oder Lösungen auch durch Lösen oder Suspendieren wenigstens eines ligninhaltigen Materials hergestellt werden. Das ligninhaltige Material enthält vorzugsweise wenigstens 10 Gew.-%, insbesondere wenigstens 15 Gew.-% und insbesondere bevorzugt wenigstens 20 Gew.-% Lignin bezogen auf das Gesamtgewicht des ligninhaltigen Materials. Das ligninhaltige Material ist vorzugsweise ausgewählt unter Stroh, Bagasse, Kraft-Lignin, Ligninsulphonat, oxidiertem Lignin, Organosolv-Lignin oder anderen ligninhaltigen Rückständen aus der Papierindustrie oder Faserherstellung, insbesondere unter Kraft-Lignin, Ligninsulfonat und oxidiertem Lignin, das bei einer elektrochemische Oxidation von nicht oxidiertem Lignin anfällt.

In einer bevorzugten Ausführungsform wird oxidiertes Lignin eingesetzt, das aus einem vorherigen Elektrolysezyklus stammt. Dabei hat es sich als vorteilhaft erwiesen, oxidiertes Lignin in wenigstens einem weiteren Elektrolysezyklus, bevorzugt in wenigstens zwei weiteren Elektrolysezyklen und insbesondere in wenigstens drei weiteren Elektrolysezyklen, einzusetzen. Vorteilhaft an dieser wiederholten Verwendung des oxidierten Lignins ist, dass wiederholt Vanillin gewonnen werden kann. Somit wird die Ausbeute an Vanillin bezogen auf die ursprünglich eingesetzte Ligninmenge deutlich gesteigert und daher die Wirtschaftlichkeit des Gesamtverfahrens erhöht. Weiterhin kann durch die wiederholte Verwendung des oxidierten Lignins die Konzentration des oxidationsempfindlichen Vanillins im Elektrolyten pro Oxidationsvorgang so gering gehalten werden, dass die unerwünschten Nebenreaktionen wie Überoxidation wirkungsvoll zurückgedrängt werden können, während die Gesamtausbeute an Vanillin über den Gesamtprozess (mehrere Elektrolysezyklen) zunimmt.

Zur Verbesserung der Löslichkeit des Lignins in der wässrigen, ligninhaltigen Suspension oder Lösung kann es vorteilhaft sein das ligninhaltige Material zusammen mit anorganischen Basen zu lösen oder zu suspendieren. Als anorganische Basen können Alkalimetallhydroxide wie NaOH oder KOH, Ammonium-Salze wie Ammoniumhydroxid und Alkalimetallcarbonate wie Natriumcarbonat, z.B. in Form von Soda, verwendet werden. Bevorzugt sind Alkalimetallhydroxide, insbesondere NaOH und KOH. Die Konzentration an anorganischen Basen in der wässrigen, ligninhaltigen Suspension oder Lösung sollte 5 mol/l und insbesondere 4 mol/l nicht überschreiten und liegt dann typischerweise im Bereich von 0,01 bis 5 mol/l, insbesondere im Bereich von 0,1 bis 4 mol/l.

Besonders bevorzugt ist es Abwässerströme oder Rückstände aus der Papier- und Zellstoffherstellung, insbesondere Schwarzlauge oder Kraft-Lignin, einzusetzen.

Bei hohen Ligninkonzentrationen in der wässrigen, ligninhaltigen Suspension oder Lösung kann sich die Viskosität der Lösung bzw. Suspension stark erhöhen und die Löslichkeit des Lignins sehr gering werden. In diesen Fällen kann es vorteilhaft sein, vor der elektrochemischen Oxidation eine Vorhydrolyse des Lignins durchzuführen, wodurch die Löslichkeit des Lignins verbessert und die Viskosität der wässrigen, ligninhaltigen Suspension oder Lösung verringert wird. Typischer Weise wird für die Vorhydrolyse von Lignin dieses in einer wässrigen Alkalimetall-Hydroxid-Lösung auf über 100 °C erhitzt. Die Konzentration des Alkalimetall-Hydroxids liegt in der Regel im Bereich von von 0,1 bis 5 mol/l, vorzugsweise bei 0,5 bis 5 mol/l, insbesondere bei 1,0 bis 3,5 mol/l. Bevorzugt wird Natriumhydroxid oder Kaliumhydroxid verwendet. In einer bevorzugten Ausführungsform des Vorhydrolyseverfahrens wird die ligninhaltige Alkalimetall-Hydroxid-Lösung auf eine Temperatur von 150 bis 250 °C, insbesondere 170 bis 190 °C erhitzt und für 1 bis 10 h, vorzugweise 2 bis 4 h stark gerührt. Das vorhydrolysierte Lignin kann vor der elektrochemischen Oxidation aus der Alkalimetall-Hydroxid-Lösung abgetrennt werden. Alternativ besteht die Möglichkeit die elektrochemische Oxidation direkt mit der ligninhaltigen Alkalimetall-Hydroxid-Lösung durchzuführen.

Das erfindungsgemäße Verfahren ermöglicht es grundsätzlich sowohl im sauren als auch im alkalischen pH-Bereich zu arbeiten. Im erfindungsgemäßen Verfahren weist die wässrige, ligninhaltige Suspension oder Lösung im Allgemeinen einen pH-Wert im Bereich von pH 0 bis 14, häufig im Bereich von pH 6 bis 14, vorzugsweise im Bereich von pH 7 bis 13, insbesondere im Bereich von pH 8 bis 13 auf.

Wie zuvor erläutert, ist das bei der Elektrolyse gebildete Vanillin unter alkalischen Bedingungen empfindlich gegenüber Oxidations- und Disproportionierungsprozessen. Daher ist es grundsätzlich für die Stabilität des erhaltenen Vanillins von Vorteil, bei niedrigen pH-Werten zu arbeiten. Da die Löslichkeit des Lignins und vielen seiner Derivate im Alkalischen am höchsten ist, kann es trotz der Stabilitätsprobleme des Vanillins sinnvoll sein, im alkalischen Bereich zu arbeiten. Aufgrund der Verwendung von Silberelektroden ist es jedoch möglich, sehr viel mildere Elektrolysebedingungen als im Stand der Technik anzuwenden, so dass ein Abbau des Vanillins nur in geringerem Maße auftritt oder gar vermieden werden kann.

In einer ersten Ausführungsform des erfindungsgemäßen Verfahrens weist die wässrige, ligninhaltige Suspension oder Lösung einen pH-Wert von pH 0 bis pH 8, vorzugsweise von pH 1 bis 5, speziell pH 1 bis pH 3 auf. Bevorzugt wird der pH-Wert mit in Wasser gut löslichen anorganischen Säuren, wie Salzsäure, Schwefelsäure, Salpetersäure oder organischen Säuren wie para-Toluolsulfonsäure oder Mischungen verschiedener Säuren eingestellt. Besonders bevorzugt ist Schwefelsäure.

In einer weiteren bevorzugten Ausführungsform des erfindungsgemäßen Verfahrens, weist die wässrige, ligninhaltige Suspension oder Lösung einen pH-Wert im Bereich von pH 6 bis pH 14, vorzugsweise von pH 7 bis pH 13, insbesondere von pH 8 bis pH 13, auf.

In einer weiteren bevorzugten Ausführungsform des erfindungsgemäßen Verfahrens, weist die wässrige, ligninhaltige Suspension oder Lösung einen pH-Wert von wenigstens pH 8, insbesondere wenigstens pH 10 und speziell wenigstens pH 12 auf, z.B. einen pH-Wert im Bereich von pH 8 bis pH 14, vorzugsweise von pH 10 bis pH 14, insbesondere von pH 12 bis pH 14.

Der wässrigen, ligninhaltigen Suspension oder Lösung werden dabei zur Verbesserung der Löslichkeit des Lignins als Additiv Alkalimetallhydroxide, insbesondere NaOH oder KOH zugegeben. Die Konzentration der Alkalimetallhydroxide liegt in der Regel in einem Bereich von 0,1 bis 5 mol/l, häufig im Bereich von 0,5 bis 5 mol/l, bevorzugt 1 bis 3,5 mol/l, insbesondere 1,0 bis 3,0 mol/l. Einige Abwasserströme der Papier- und Zellstoffherstellung, wie z.B. Schwarzlauge, weisen schon produktionsbedingt eine entsprechende Konzentration an Alkalimetallhydroxiden auf.

Die wässrige, ligninhaltige Suspension oder Lösung kann zur Verbesserung der Leitfähigkeit ein Leitsalz enthalten. Hierbei handelt es sich im Allgemeinen um Alkalimetallsalze wie Salze von Li, Na, K oder quaternäre Ammoniumsalze wie Tetra(C₁-C₆-alkyl)ammonium oder Tri(C₁-C₆-alkyl)methylammoniumsalze. Als Gegenionen kommen Sulfat, Hydrogensulfat, Alkylsulfate, Arylsulfate, Halogenide, Phosphate, Carbonate, Alkylphosphate, Alkylcarbonate, Nitrat, Alkoholate, Tetrafluorborat, Hexafluorphosphat, Perchlorat oder Bistriflat- oder Bistriflimid in Betracht.

Daneben eignen sich als Leitsalze auch ionische Flüssigkeiten ("Ionic Liquids"). Geeignete elektrochemisch stabile ionische Flüssigkeiten sind beschrieben in "Ionic Liquids in Synthesis", Hrsg. Peter Wasserscheid, Tom Welton, Verlag Wiley-VCH 2003, Kap. 1 bis 3.

Für die elektrochemische Oxidation des Lignins kann der wässrigen, ligninhaltigen Suspension oder Lösung ein metallhaltiger oder metallfreier Mediator zugegeben werden. Unter Mediatoren werden Redoxpaare verstanden, die eine indirekte elektrochemische Oxidation ermöglichen. Der Mediator wird elektrochemisch in die höhere Oxidationsstufe überführt, wirkt dann als Oxidationsmittel und regeneriert sich danach wieder durch elektrochemische Oxidation. Es handelt sich daher um eine indirekte elektrochemische Oxidation der organischen Verbindung, da der Mediator das Oxidationsmittel ist. Die Oxidation der organischen Verbindung mit dem Mediator in der oxidierten Form kann dabei in der Elektrolysezelle ausgeführt werden, in der der Mediator in die oxidierte Form überführt wurde, oder in einem oder mehreren separaten Reaktoren ("ex-cell-Verfahren"). Die letztgenannte Methode hat den Vorteil, dass eventuelle zurückgebliebene Spuren der zu oxidierenden organischen Verbindung bei der Herstellung oder Regeneration des Mediators nicht stören.

Geeignete Mediatoren sind Verbindungen, die in zwei Oxidationsstufen vorliegen können, in der höheren Oxidationsstufe als Oxidationsmittel wirken und elektrochemisch regenerierbar sind. Als Mediatoren können z.B. Salze oder Komplexe folgender Redoxpaare zur Anwendung kommen: Ce (III/IV), Cr (II/III), Cr (III/VI), Ti (II/III), V (II/III), V (III/IV), V (IV/V), Ag (I/II), AgO⁺/AgO⁻, Cu (I/II), Sn (II/IV), Co (II/III), Mn (II/III), Mn (II/IV), Os (IV/VIII), Os (III/IV), Br₂/Br⁻/BrO₃, I-/I₂, I₃⁺/I₂ IO₃⁺/IO₄⁻, Fremys Salz (Dikaliumnitrosodisulfonat) oder auch organische Mediatoren, wie ABTS (2,2'-Azino-bis-(3-ethylbenzothiazolin-6-sulfonsäure), TEMPO, Viologene wie Violursäure, NAD⁺/NADH, NADP⁺/NADPH, wobei es sich bei den angegebenen Systemen auch um Metallkomplexe mit diversen Liganden oder auch Lösungsmittel-Liganden handeln kann, wie z.B. H₂O, NH₃, CN-, OH-, SCN-, Halogene, O₂, Acetylacetonat, Dipyridyl, Phenanthrolin oder 1 ,10-Phenanthrolin 5,6-dion. Bevorzugt werden in dem erfindungsgemäßen Verfahren übergangsmetallfreie Mediatoren, z.B. Nitrosodisulfonate wie Fremys Salz (Dikaliumnitrosodisulfonat), eingesetzt. Der Mediator wird bevorzugt in Mengen von 0,1 bis 30 Gew. %, besonders bevorzugt von 1 bis 20 Gew. %, bezogen auf das Gesamtgewicht der wässrigen, ligninhaltigen Suspension oder Lösung eingesetzt.

In einer besonders bevorzugten Ausführungsform wird das erfindungsgemäße Verfahren ohne Zusatz von Mediatoren ausgeführt.

Die wässrige, ligninhaltige Suspension oder Lösung kann weiterhin ein inertes Lösungsmittel enthalten. Geeignete Lösungsmittel sind polar-aprotische Lösungsmittel mit hoher elektrochemischer Stabilität wie Acetonitril, Propionitril, Adiponitril, Korksäuredinitril, Propylencarbonat, Ethylencarbonat, Dichlormethan, Nitromethan, Chloroform, Tetrachlorkohlenstoff, 1,2- Dichlorethan, 1,1,2,2-Tetrachlorethan, Trichlorethylen, Tetrachlorethylen, Hexafluoraceton, N-Methylpyrrolidon, Hexamethylphosphorsäuretriamid, Dimethylsulfoxid und Dimethylpropylenharnstoff (DMPU). Weitere geeignete polar-aprotische Lösungsmittel sind beschrieben in Kosuke Izutsu, "Electrochemistry in Nonaqueous Solutions", Verlag Wiley-VCH 2002, Kap. 1.

In dem erfindungsgemäßen Verfahren werden im Allgemeinen inerte Lösungsmittel in einer Menge von nicht mehr als 60 Gew.-%, vorzugsweise nicht mehr als 30 Gew.-%, insbesondere nicht mehr als 20 Gew.-%, z.B. 2,5 bis 30 Gew.-% oder 5 bis 20 Gew.-%, bezogen auf die Gesamtmenge der eingesetzten wässrigen, ligninhaltigen Suspension oder Lösung, eingesetzt.

Das nach dem erfindungsgemäßen Verfahren erhaltene Vanillin kann aus der wässrigen, ligninhaltigen Lösung durch dem Fachmann bekannte Methoden entfernt werden. Bevorzugt wird das Vanillin durch Destillation oder Extraktion der wässrigen, ligninhaltigen Suspension oder Lösung entzogen.

Als destillative Methoden eignen sich dem Fachmann bekannte Destillationsprozesse, wie z.B. Vakuumdestillation, Destillation unter Schutzgasatmosphäre oder Wasserdampfdestillation. Ein Vorteil der Vanillinabtrennung über destillative Prozesse ist es, dass das Vanillin nicht mit potentiell gesundheitsgefährdenden organischen Lösungsmitteln in Kontakt gebracht wird.

Vanillin kann ebenso durch Extraktion aus der wässrigen, ligninhaltigen Suspension oder Lösung entfernt werden. Dies ist besonders vorteilhaft, da das empfindliche Vanillin keiner weiteren thermischen Belastung ausgesetzt wird. Hierfür eignen sich dem Fachmann bekannte Extraktionsprozesse.

Die wässrige, ligninhaltige Suspension oder Lösung kann mit einem organischem Lösungsmittel versetzt werden, um so dass gebildete Vanillin abzutrennen (flüssig-flüssig Extraktion). Als organische Lösungsmittel eignen sich mit Wasser nicht mischbare organische Lösungsmittel z.B. Kohlenwasserstoffe mit 5 bis 12 Kohlenstoffatomen wie Hexan oder Oktan, chlorierte Kohlenwasserstoffe mit 1 bis 10 Kohlenstoffatomen wie Dichlormethan oder Chloroform, aliphatische Ether mit 2 bis 10 Kohlenstoffatomen wie Diethylether oder Diisopropylether, zyklische Ether oder aliphatische Ester wie Ethansäureethylester. Bevorzugt sind halogenfreie organische Lösungsmittel. Weiterhin ist es möglich Vanillin mit Hilfe überkritischer Fluids zu extrahieren. Hierfür eignet sich insbesondere überkritisches CO₂.

Das gebildete Lignin kann ebenso durch Festphasenextraktion aus der wässrigen, ligninhaltigen Suspension oder Lösung entfernt werden. Hierfür werden Festphasenextraktionsmittel zu der wässrigen, ligninhaltigen Suspension oder Lösung gegeben. Das an das Extraktionsmittel adsorbierte Vanillin (Vanillat) kann anschließend mit dem Fachmann bekannten polaren, organischen Lösungsmitteln wie z.B. Methanol von der festen Phase eluiert werden. Weiterhin ist auch eine Festphasenextraktion analog zur Festphasensynthese möglich. In diesem Fall wird das Vanillin kovalent als Vanillat an die feste Phase gebunden. Nach Abtrennung der festen Phasen von der wässrigen, ligninhaltigen Suspension oder Lösung wird das Vanillin durch Lösen der kovalenten Bindung wieder freigesetzt. In beiden Fällen wird ein aufkonzentriertes Rohprodukt erhalten, welches dann durch Destillation einfacher gereinigt und isoliert werden kann.

In einer bevorzugten Ausführungsform des erfindungsgemäßen Verfahrens wird das erzeugte Vanillin durch Festphasenextraktion aus der wässrigen, ligninhaltigen Lösung oder Suspension entfernt.

Weiterhin ist es möglich die wässrige, ligninhaltige Suspension oder Lösung vor der Abtrennung des Vanillins von den flüchtigen Bestandteilen der Lösung oder Suspension destillativ zu befreien. Aus dem verbleibenden Rückstand kann anschließend das Vanillin mit Hilfe der zuvor genannten Extraktionsmittel extrahiert werden.

Die Abtrennung des Vanillins kann kontinuierlich oder diskontinuierlich erfolgen. Besonders vorteilhaft ist es das Vanillin während der elektrochemischen Oxidation kontinuierlich aus der wässrigen, ligninhaltigen Suspension oder Lösung zu entfernen. Insbesondere ist es bevorzugt das Vanillin durch kontinuierliche (Festphasen-) Extraktion oder Wasserdampfdestillation aus der wässrigen, ligninhaltigen Lösung zu entfernen.

Überoxidationsprodukte des Vanillins, die bei der Elektrolyse gebildet werden können, lassen sich leicht entfernen. Untersuchungen der Erfinder haben gezeigt, dass Überoxidationsprodukte, die an einer erfindungsgemäß verwendeten Silberelektrode gebildet wurden, einen hohen Anteil an Carboxyl-Gruppen aufweisen, so dass sie in einfacher Weise durch dem Fachmann bekannte Techniken wie Verwendung von Ionentauscher oder Extraktion aus dem Reaktionsprodukt entfernt werden können.

Gemäß dem erfindungsgemäßen Verfahren wird das Vanillin ohne Einsatz einer Schwermetall-Anode hergestellt. Daher ist aufgrund der geringen Schwermetallbelastung des hergestellten Vanillins dieses in der Lebensmittelindustrie einsetzbar. Ein weiterer Gegenstand der Erfindung ist somit die Verwendung des Vanillins, welches durch das beschriebene Verfahren hergestellt wurde, als Aromastoff in der Lebensmittelindustrie einzusetzen.

Nach Beendigung der Elektrolyse enthält die wässrige, ligninhaltige Suspension oder Lösung neben dem gebildeten Vanillin noch oxidiertes Lignin. Nach Abtrennung des Vanillins und gegebenenfalls anderer niedermolekularer Produkte kann das oxidierte Lignin durch Trocknung der wässrigen, ligninhaltigen Lösung gewonnen werden. Ein so hergestelltes Lignin kann beispielsweise vorteilhaft als Additiv in der Baustoffindustrie eingesetzt werden, beispielsweise als Zement- oder Beton-Additiv.

Die folgenden Beispiele sollen die Erfindung weiter beschreiben und sind nicht einschränkend zu verstehen.

### Analytik

Für die gaschromatographische Analyse der Elektrolyseprodukte wurde als stationäre Phase eine HP-5 Säule der Fa. Agilent mit 30 m Länge, 0,25 mm Durchmesser und 1 µm Schichtdicke verwendet. Diese Säule wird mittels Temperaturprogramm von 50 °C innerhalb von 10 min mit 10 °C/min auf 290 °C aufgeheizt. Diese Temperatur wird für 15 min gehalten. Als Trägergas wurde Wasserstoff mit einer Durchflussgeschwindigkeit von 46,5 mL/min verwendet.

### Beispiel 1:

520 mg Kraft-Lignin wurden in einem Elektrolyten aus 81 g 3 M wässriger NaOH in einer ungeteilten Zelle unter Rühren gelöst. Die Zelle weist eine Anode aus Silberblech und eine Kathode aus Nickelblech auf (je 2,5 cm×3 cm), die parallel zueinander im Abstand von 0,5 cm angebracht sind. Die Lösung wurde bei einer Stromdichte von 1,9 mA/cm² und einer Temperatur von 80 °C unter Rühren für 28 Stunden (Q = 1411 C) elektrolysiert. Die sich einstellende Zellspannung lag im Bereich von 2-3 V. Nach durchflossener Ladungsmenge wurde der Zellinhalt auf Raumtemperatur abgekühlt und mit einer bekannten Menge eines Standards (n-Hexadecan) versetzt. Anschließend wurde von eventuell vorhandenem Feststoff filtriert. Dann wurde die Lösung mit 10 %-iger, wässriger Salzsäure auf pH∼1-2 eingestellt und mit 20 mL Dichlormethan versetzt. Der ausgefallene, gallertartige Feststoff wurde über Kieselgur filtriert und mit Dichlormethan nachgewaschen. Die organische Phase wird abgetrennt. Die wässrige Phase wurde noch dreimal mit je 80 mL Dichlormethan extrahiert. Die vereinigten organischen Phasen wurden mit 50 mL Wasser und 50 mL gesättigter Kochsalzlösung gewaschen, bevor sie im Anschluss über Na₂SO₄ getrocknet wurden. Nach dem Entfernen des Lösungsmittels unter vermindertem Druck bleibt ein öliger, goldbrauner Rückstand zurück der bezüglich seiner Zusammensetzung gaschromatographisch analysiert wurde.

Die gaschromatographische Analyse des organischen Rohproduktes ergab folgende typische Zusammensetzung, bezogen auf eingesetztes Lignin (Gew.-%):
1,20 % Vanillin, 0,66 % Acetovanillon, 0,21 % Vanillinsäure. Damit liegt die Selektivität für Vanillin bei 58%.

### Beispiel 2

Die Durchführung der Elektrolyse erfolgte analog Beispiel 1 mit folgender Änderung: Die Lösung wurde 20 Stunden elektrolysiert (Q = 1000 C). Typische Zusammensetzung der organischen Extrakte, bezogen auf eingesetztes Lignin (Gew.-%):
1,04 % Vanillin, 0,56 % Acetovanillon, 0,25 % Vanillinsäure. Damit ergibt sich eine Selektivität für Vanillin von 56,2%.

### Vergleichsbeispiel

Die Durchführung erfolgte analog Beispiel 1 mit folgender Änderung: Die Lösung wurde 22 Stunden (Q = 1411 C) unter Verwendung einer Ni-Anode und einer Ni-Kathode elektrolysiert. Typische Zusammensetzung der organischen Extrakte, bezogen auf eingesetztes Lignin (Gew.-%): 0,57 % Vanillin, 0,09 % Acetovanillon.

### Beispiel 3

8,336 g Kraft-Lignin wurden in einer temperierbaren Zelle mit Kühlmantel vorgelegt und unter Rühren in 1008 g 3 M wässriger NaOH gelöst. In der Elektrolyseanordnung wurden 11 Silberbleche (je 6,5 cm×7,0 cm) bipolar im Abstand von 0,3 cm geschaltet, so dass die Zelle aus zehn Halbräumen bestand. Die Elektrolyse erfolgte galvanostatisch bei einer Stromdichte von j = 1.9 mA/cm² und einer Temperatur von 80 °C. Die Lösung wurde 12,6 Stunden (Q = 4000 C; auf Elektrolyt bezogen: Q = 40000 C) elektrolysiert. Die sich einstellende Zellspannung lag im Bereich von 3-3,5 V. Nach der durchflossenen Ladungsmenge wurde der Zellinhalt auf Raumtemperatur gebracht und über eine Fritte von vorhandenem Feststoff abfiltriert. Die Filtrate wurden mit 10 %-iger, wässriger Salzsäure auf pH = 1-2 eingestellt und mit 100 mL Dichlormethan versetzt. Der ausgefallene, gallertartige Feststoff wurde über Kieselgur filtriert und mit Dichlormethan nachgewaschen.

Die organische Phase des Filtrats wurde abgetrennt und die wässrige Phase in zwei Portionen je viermal mit je 100 mL Dichlormethan extrahiert. Die vereinigten organischen Phasen wurden mit 200 mL gesättigter Kochsalzlösung gewaschen, bevor sie im Anschluss über Na₂SO₄ getrocknet wurden. Nach dem Entfernen des Lösungsmittels unter vermindertem Druck bleibt ein öliger, goldbrauner Rückstand zurück, der säulenchromatographisch (Kieselgel 60, Cyclohexan-Essigester-Gradient v/v 3:2 → 1:1) gereinigt wurde.

Die säulenchromatographische Reinigung des organischen Rohproduktes (m = 191 mg) ergab folgende typische Ausbeute, bezogen auf eingesetztes Lignin: Durchlauf 1: 15 mg = 0,18 % Guajakol; 45 mg = 0,54 % Vanillin; 20 mg = 0,24 % Acetovanillon.

Der Lignin-Rückstand in der Fritte wurde durch Zugabe von 1008 g 3 M NaOH vom Kieselgur gelöst. Nach Filtration wurde die Lösung erneut unter den oben genannten Bedingungen elektrolysiert, aufgearbeitet und charakterisiert. Die säulenchromatographische Reinigung des organischen Rohproduktes (m = 76 mg) ergab folgende typische Ausbeuten, bezogen auf eingesetztes Lignin (Gew.-%):
Durchlauf 2: 39 mg = 0,47 % Vanillin.

### Beispiel 4:

523 mg Kraft-Lignin wurden in einem Elektrolyten aus 80 g 1 M wässriger NaOH gelöst. in einer temperierbaren, ungeteilten Zelle unter Rühren gelöst. Die Zelle wies zwei Elektroden aus Silberblech auf (je 2,5 cm×3,2 cm), die parallel zueinander im Abstand von 0,5 cm angebracht waren. Die Lösung wurde bei einer Stromdichte von 1,9 mA/cm² und einer Temperatur von 80°C für 24,5 Stunden (Q = 1411 C) elektrolysiert. Nach durchflossener Ladungsmenge wurde der Zellinhalt auf Raumtemperatur abgekühlt und mit einer bekannten Menge eines Standards (n-Hexadecan) versetzt. Anschließend wurde die Lösung mit 10%iger, wässriger Salzsäure auf pH = 1-2 eingestellt und mit 20 mL Dichlormethan versetzt. Der ausgefallene, gallertartige Feststoff wurde über Kieselgur filtriert und mit Dichlormethan nachgewaschen. Die organische Phase wurde abgetrennt. Die wässrige Phase wurde noch dreimal mit je 80 mL Dichlormethan extrahiert. Die vereinigten organischen Phasen wurden mit 50 mL Wasser und 50 mL gesättigter Kochsalzlösung gewaschen, bevor sie im Anschluss über Na₂SO₄ getrocknet wurden. Nach dem Entfernen des Lösungsmittels unter vermindertem Druck bleibt ein öliger, goldbrauner Rückstand (m = 15 mg) zurück, der bezüglich seiner Zusammensetzung gaschromatographisch analysiert wurde.

Die gaschromatographische Analyse des organischen Rohproduktes ergab folgende typische Ausbeuten, bezogen auf eingesetztes Lignin (Gew.-%): 0,65 % Vanillin, 0,12 % Acetovanillon.

### Beispiel 5

Die Durchführung der Elektrolyse erfolgte analog Beispiel 4 mit folgender Änderung: 526 mg Kraft-Lignin wurden in einem Elektrolyten aus 80 g 0,5 M wässriger NaOH unter Rühren gelöst und bei einer Stromdichte von 1,9 mA/cm² und einer Temperatur von 80°C für 20,6 Stunden (Q = 1411 C) elektrolysiert. Typische Zusammensetzung der organischen Extrakte (m = 57 mg), bezogen auf eingesetztes Lignin (Gew.-%): 1,37 % Vanillin, 0,10 % Acetovanillon.

### Beispiel 6:

Die Durchführung der Elektrolyse erfolgte analog Beispiel 4 mit folgender Änderung: 525 mg Alkali-Lignin wurden in einem Elektrolyten aus 86 g 3 M NaOH unter Rühren gelöst und bei einer Stromdichte von 1,9 mA/cm² und einer Temperatur von 80°C für 20,6 Stunden (Q = 1411 C) elektrolysiert. Es wurden zwei Silberbleche (4,0 cm×2,5 cm) im Abstand von 0,5 cm als Elektroden verwendet. Typische Ausbeuten der organischen Extrakte (m = 41 mg), bezogen auf eingesetztes Lignin (Gew.-%): 0,76 % Vanillin, 0,37 % Acetovanillon, 0,88 % Vanillinsäure.

### Vergleichsbeispiel 2:

Die Durchführung der Elektrolyse erfolgte analog Beispiel 4 mit folgender Änderung: Die Zelle wies zwei Elektroden aus Nickelblech auf (je 2,5 cm×4,0 cm), die parallel zueinander im Abstand von 0,5 cm angebracht waren.

525 mg Alkali-Lignin wurden in einem Elektrolyten aus 80 g 1 M NaOH unter Rühren gelöst und bei einer Stromdichte von 1,9 mA/cm² und einer Temperatur von 80°C für 23,1 Stunden (Q = 1411 C) elektrolysiert. Typische Ausbeute der organischen Extrakte (m = 38 mg), bezogen auf eingesetztes Lignin (Gew.-%): 0,38 % Vanillin.

### Beispiele 7 bis 9:

524-526 mg Kraft-Lignin wurden in 80 g Elektrolyt in einer temperierbaren, ungeteilten Zelle unter Rühren gelöst. Die Zelle wies eine Anode aus Ag/Ni-Legierung (0.5 cm×32.5 cm) auf, die spiralförmig in der Zelle befestigt wurde. Die Legierung bestand zu 90 % aus Silber und 10 % Nickel. Als Kathode diente ein Nickelnetz, das mittig in der Spirale in den Elektrolyten eintauchte. Die Lösung wurde bei einer Stromdichte von 1,9 mA/cm² und einer Temperatur von 80°C (Q = 1411 C) für 12,6 Stunden elektrolysiert. Die maximale Klemmspannung während der Reaktion betrug 3.0 V. Nach durchflossener Ladungsmenge wurde der Zellinhalt auf Raumtemperatur abgekühlt, mit einer bekannten Menge eines Standards (n-Hexadecan) versetzt und von eventuell vorhandenem Feststoff filtriert. Anschließend wurde die Lösung mit konzentrierter Salzsäure auf pH = 1-2 eingestellt und mit 20 mL Dichlormethan versetzt. Der ausgefallene, gallertartige Feststoff wurde über Kieselgur filtriert und mit ca. 25 ml Dichlormethan nachgewaschen. Die organische Phase wurde abgetrennt. Die wässrige Phase wurde noch dreimal mit je 80 mL Dichlormethan extrahiert. Die vereinigten organischen Phasen werden mit 50 mL gesättigter Kochsalzlösung gewaschen, bevor sie im Anschluss über Na₂SO₄ getrocknet werden. Nach dem Entfernen des Lösungsmittels unter vermindertem Druck verblieb ein öliger, meist goldbrauner Rückstand zurück, der bezüglich seiner Zusammensetzung gaschromatographisch analysiert wurde. Die gaschromatographische Analyse der organischen Rohprodukte ergab typische Zusammensetzungen, bezogen auf eingesetztes Lignin (Gew%), die in Tabelle 1 zusammengefasst sind.

**Tabelle 1:**

| Beispiel | Elektrolyt | Ausbeute [Gew.-%] ¹⁾ | | | |
|---|---|---|---|---|---|
| | | Vanillin | Acetovanillin | Gujakol | Vanillinsäure |
| 7 | 3 M NaOH | 1.61 | 0.36 | 0.09 | 0.27 |
| 8 | 2 M NaOH | 1.51 | 0.42 | - | 0.23 |
| 9 | 1 M NaOH | 2,84 | 0.04 | - | -- |

| | | | | | |
|---|---|---|---|---|---|
| ^{[1]} Bestimmung mittels mittels Gaschromatographie gegen internen Standard, bezogen auf eingesetztes Kraft-Lignin (Gew%). | | | | | |

### Beispiel 10:

Die Durchführung erfolgte analog zu Beispiel 7 mit folgender Variation: 525-526 mg Kraft-Lignin wurden in 85 g 3 M wässriger NaOH in einer ungeteilten Zelle unter Rühren gelöst. Die Zelle ist versehen mit Anode und Kathode bestehend aus Cuprosilber (3,0×4,0 cm²), die parallel zueinander im Abstand von 0,5 cm angebracht waren. Die Lösung wurde für 17,2 h (Q = 1411 C) elektrolysiert. Die maximale Zellspannung während der Reaktion betrug 2,9 V.

Die Ausbeuten der organischen Extrakte, bezogen auf eingesetztes Lignin (Gew.-%) betrugen: 1,51 % Vanillin, 0,15 % Acetovanillon.

## Patentansprüche

1. Verfahren zur Herstellung von Vanillin, umfassend eine elektrochemische Oxidation einer wässrigen, ligninhaltigen Suspension oder Lösung an einer Anode, wobei als Anode eine Silberelektrode verwendet wird.

2. Verfahren gemäß Anspruch 1, in dem als Silberelektrode eine Elektrode eingesetzt wird, in der Silber oder eine silberhaltige Legierung als Beschichtung auf einem elektrisch leitfähigen, von Silber verschiedenen Träger angeordnet ist.

3. Verfahren gemäß Anspruch 2, in dem Streckmetalle oder Bleche als Träger der Silberbeschichtung verwendet werden.

4. Verfahren gemäß einem der vorhergehenden Ansprüche, in dem die als Kathode eingesetzte Elektrode eine Oberfläche aufweist, die ausgewählt ist unter Silber, Nickel, silberhaltigen Legierungen, RuOₓTiOₓ-Mischoxiden, platiniertem Titan, Platin, Edelstahl, Graphit oder Kohle.

5. Verfahren gemäß einem der vorhergehenden Ansprüche, in dem die Elektrolyse mit einer Stromdichte in einem Bereich von 1 bis 1000 mA/cm² durchgeführt wird.

6. Verfahren gemäß einem der vorhergehenden Ansprüche, in dem die elektrochemische Oxidation bei Temperaturen in einem Bereich von 0 bis 100 °C durchgeführt wird.

7. Verfahren gemäß einem der vorhergehenden Ansprüche, in dem die elektrochemische Oxidation bei Drücken unterhalb 1000 kPa durchgeführt wird.

8. Verfahren gemäß einem der vorhergehenden Ansprüche, in dem als wässrige, ligninhaltige Suspension oder Lösung ein ligninhaltiger Strom aus der Papierstoff-, Zellstoff oder Celluloseherstellung verwendet wird.

9. Verfahren gemäß einem der Ansprüche 1 bis 7, in dem die ligninhaltige Suspension oder Lösung durch Lösen oder Suspendieren wenigstens eines ligninhaltigen Materials hergestellt wird, das ausgewählt ist unter Lignin aus Schwarzlauge, Kraft-Lignin, Ligninsulfonat, Alkalilignin, Organosolv-Lignin und entsprechenden Rückständen aus der Papierindustrie, Zellstoff- oder Celluloseherstellung.

10. Verfahren gemäß einem der vorhergehenden Ansprüche, in dem die wässrige, ligninhaltige Suspension oder Lösung 0,5 bis 30 Gew.-% Lignin oder ein Derivat des Lignins, bezogen auf das Gesamtgewicht der wässrigen, ligninhaltigen Suspension oder Lösung, enthält.

11. Verfahren gemäß einem der vorhergehenden Ansprüche, in dem die wässrige, ligninhaltige Suspension oder Lösung durch Lösen oder Suspendieren von oxidiertem Lignin hergestellt wird, das durch das Verfahren gemäß einem der vorhergehenden Ansprüche erhalten wurde.

12. Verfahren gemäß einem der vorhergehenden Ansprüche, in dem die wässrige, ligninhaltige Suspension oder Lösung einen pH in einem Bereich von pH 8 bis pH 14 aufweist.

13. Verfahren gemäß einem der vorhergehenden Ansprüche, wobei das bei der Oxidation gebildete Vanillin kontinuierlich aus der wässrigen, ligninhaltigen Lösung oder Suspension entfernt wird.

14. Verfahren gemäß Anspruch 13, wobei das bei der elektrochemischen Oxidation gebildete Vanillin durch kontinuierliche Extraktion mit einem organischen Lösungsmittel aus der wässrigen, ligninhaltigen Lösung oder Suspension entfernt wird.

15. Verfahren gemäß einem der vorhergehenden Ansprüche, in dem das Vanillin durch Festphasenextraktion aus der wässrigen, ligninhaltigen Lösung oder Suspension entfernt wird.

## Claims

1. A method for producing vanillin, which comprises an electrochemical oxidation of an aqueous, lignin-comprising suspension or solution at an anode, wherein the anode used is a silver electrode.

2. The method according to claim 1, in which, as silver electrode, an electrode is used in which silver or a silver-comprising alloy is arranged as coating on an electrically conducting support that is different from silver.

3. The method according to claim 2, in which expanded metals or metal sheets are used as supports of the silver coating.

4. The method according to any one of the preceding claims, in which the electrode used as cathode has a surface which is selected from silver, nickel, silver-comprising alloys, RuOₓTiOₓ mixed oxides, platinated titanium, platinum, stainless steel, graphite or carbon.

5. The method according to any one of the preceding claims, in which the electrolysis is carried out at a current density in a range from 1 to 1000 mA/cm².

6. The method according to any one of the preceding claims, in which the electrochemical oxidation is carried out at temperatures in a range from 0 to 100°C.

7. The method according to any one of the preceding claims, in which the electrochemical oxidation is carried out at pressures below 1000 kPa.

8. The method according to any one of the preceding claims, in which the aqueous, lignin-comprising suspension or solution used is a lignin-comprising stream from the production of paper pulp, pulp or cellulose.

9. The method according to any one of claims 1 to 7, in which the lignin-comprising suspension or solution is produced by dissolving or suspending at least one lignin-comprising material which is selected from lignin from black liquor, kraft lignin, lignosulfonate, alkali lignin, organosolv lignin and corresponding residues from the paper industry, pulp production or cellulose production.

10. The method according to any one of the preceding claims, in which the aqueous lignin-comprising suspension or solution comprises 0.5 to 30% by weight of lignin or a derivative of lignin, based on the total weight of the aqueous, lignin-comprising suspension or solution.

11. The method according to any one of the preceding claims, in which the aqueous lignin-comprising suspension or solution is produced by dissolving or suspending oxidized lignin that was obtained by the method according to any one of the preceding claims.

12. The method according to any one of the preceding claims, in which the aqueous lignin-comprising suspension or solution has a pH in a range from pH 8 to pH 14.

13. The method according to any one of the preceding claims, wherein the vanillin formed in the oxidation is continuously removed from the aqueous, lignin-comprising solution or suspension.

14. The method according to claim 13, wherein the vanillin formed in the electrochemical oxidation is removed from the aqueous, lignin-comprising solution or suspension by continuous extraction with an organic solvent.

15. The method according to any one of the preceding claims, in which the vanillin is removed from the aqueous, lignin-comprising solution or suspension by solid-phase extraction.

## Revendications

1. Procédé de fabrication de vanilline, comprenant une oxydation électrochimique d'une suspension ou d'une solution aqueuse contenant de la lignine sur une anode, une électrode en argent étant utilisée en tant qu'anode.

2. Procédé selon la revendication 1, dans lequel une électrode dans laquelle de l'argent ou un alliage contenant de l'argent est agencé en tant que revêtement sur un support électriquement conducteur, différent de l'argent, est utilisée en tant qu'électrode en argent.

3. Procédé selon la revendication 2, dans lequel des métaux déployés ou des tôles sont utilisés en tant que support du revêtement d'argent.

4. Procédé selon l'une quelconque des revendications précédentes, dans lequel l'électrode utilisée en tant que cathode présente une surface qui est choisie parmi l'argent, le nickel, les alliages contenant de l'argent, les oxydes mixtes RuOₓTiOₓ, le titane platiné, le platine, l'acier inoxydable, le graphite ou le charbon.

5. Procédé selon l'une quelconque des revendications précédentes, dans lequel l'électrolyse est réalisée avec une densité de courant dans la plage allant de 1 à 1 000 mA/cm².

6. Procédé selon l'une quelconque des revendications précédentes, dans lequel l'oxydation électrochimique est réalisée à des températures dans une plage allant de 0 à 100 °C.

7. Procédé selon l'une quelconque des revendications précédentes, dans lequel l'oxydation électrochimique est réalisée à des pressions inférieures à 1 000 kPa.

8. Procédé selon l'une quelconque des revendications précédentes, dans lequel un courant contenant de la lignine issu de la fabrication de pâte à papier, de pâte chimique ou de cellulose est utilisé en tant que suspension ou solution aqueuse contenant de la lignine.

9. Procédé selon l'une quelconque des revendication 1 à 7, dans lequel la suspension ou solution contenant de la lignine est fabriquée par dissolution ou suspension d'au moins un matériau contenant de la lignine, qui est choisi parmi la lignine de lessive noire, la lignine kraft, le sulfonate de lignine, la lignine alcaline, la lignine organosolv et les résidus correspondants de l'industrie du papier, de la fabrication de pâte chimique ou de cellulose.

10. Procédé selon l'une quelconque des revendications précédentes, dans lequel la suspension ou solution aqueuse contenant de la lignine contient 0,5 à 30 % en poids de lignine ou d'un dérivé de lignine, par rapport au poids total de la suspension ou solution aqueuse contenant de la lignine.

11. Procédé selon l'une quelconque des revendications précédentes, dans lequel la suspension ou solution aqueuse contenant de la lignine est fabriquée par dissolution ou suspension de lignine oxydée, qui a été obtenue par le procédé selon l'une quelconque des revendications précédentes.

12. Procédé selon l'une quelconque des revendications précédentes, dans lequel la suspension ou solution aqueuse contenant de la lignine présente un pH dans la plage allant de pH 8 à pH 14.

13. Procédé selon l'une quelconque des revendications précédentes, dans lequel la vanilline formée lors de l'oxydation est éliminée en continu de la solution ou suspension aqueuse contenant de la lignine.

14. Procédé selon la revendication 13, dans lequel la vanilline formée lors de l'oxydation électrochimique est éliminée par extraction continue avec un solvant organique de la solution ou suspension aqueuse contenant de la lignine.

15. Procédé selon l'une quelconque des revendications précédentes, dans lequel la vanilline est éliminée de la solution ou suspension aqueuse contenant de la lignine par extraction en phase solide.
